# EUROPEAN PATENT SPECIFICATION

(11) **EP 3 289 058 B1**
(45) Date of publication and mention of the grant of the patent: **24.10.2018**
(21) Application number: 16718690.7
(22) Date of filing: 26.04.2016
(51) Int. Cl.: C11D 17/00, C11D 3/37, C11D 3/22

(54) **POLYMER SHELL MICROCAPSULES WITH DEPOSITION POLYMER**
MIKROKAPSELN MIT POLYMERSCHALE MIT ABSCHEIDUNGSPOLYMER
MICROCAPSULES À ENVELOPPE DE POLYMÈRE AVEC DÉPÔT POLYMÈRE

(30) Priority: 01.05.2015 EP 15166143
(43) Date of publication of application: 07.03.2018
(73) Proprietor: Unilever PLC, London, Greater London EC4Y 0DY (GB); Unilever N.V., 3013 AL Rotterdam (NL)
(72) Inventor: FERGUSON, Paul, Wirral Merseyside CH63 3JW (GB); JONES, Christopher Clarkson, Wirral Merseyside CH63 3JW (GB)
(74) Representative: James, Helen Sarah
(86) International application number: PCT/EP2016/059321
(87) International publication number: WO 2016/177607

(56) References cited:
- WO-A1-2007/062833
- WO-A1-2013/026657
- WO-A2-2014/079745
- US-A1- 2013 330 292

## Description

### TECHNICAL FIELD

This invention relates to polymer shell microcapsules with deposition polymer for the delivery of benefit agents and their incorporation into consumer products and to a process for making the microcapsules.

### BACKGROUND

WO07062833 (Unilever) discloses in example 2 the grafting of a locust bean gum deposition polymer onto a polyurethane capsule using vinyl acetate addition polymerization. Claim 7 mentions polyxyloglucan (also called xyloglucan) as uncharged polysaccharide material and claim 3 mentions melamine formaldehyde as shell material.

WO08145547 (Unilever) discloses a process for attachment of a nonionic deposition polymer, substantive to textiles, to core shell particles. Example 7 forms melamine formaldehyde microcapsules and then attaches a locust bean gum deposition polymer to them using melamine formaldehyde pre-polymer. Example 8 attaches both locust bean gum and xyloglucan nonionic deposition polymers to microcapsules by use of various addition polymers.

WO2012/007438 (Unilever) discloses a benefit agent containing particle comprising a multi layered shell in which the outer shell may be a polyurea (see page 8 line 27) with a deposition polymer exterior to the shell (claim 4 and p28-30). The inner shell is poly methacrylate. Page 36 suggests to add deposition polymer late and, if necessary, with "further isocyanate and co-monomer". Example 1 is polyurethane. The only deposition polymer used is a PET-POET polymer chain post-grafted onto polyurethane with polyurethane (from a condensation reaction).

WO2012/038144 (Unilever) discloses in example 1 the attachment of a Xyloglucan deposition agent to preformed melamine formaldehyde perfume microcapsules by means of a pre-polymer solution of further melamine formaldehyde. The perfume deposition in the absence of mannanase enzyme was shown to be the same as locust bean gum. In the presence of mannanase the xyloglucan performed significantly better than the locust bean gum control. In example 3 the deposition agents were attached using vinyl acetate.

US2014/0142019 (Unilever) discloses a benefit agent delivery particle comprising hydroxypropylcellulose (HPC). The benefit agent delivery particle may further comprise a non-polysaccharide polymer, preferably an aminoplast polymer. The benefit agent delivery particle may comprise a perfume.

The process for the preparation of the particles is preferably a two-step process in which the first step forms a particle comprising the benefit agent and the second step applies a coating to the capsule which includes the HPC as a deposition polymer. The first step can either be step-growth or addition polymerisation and the second step (i.e. the attachment of the HPC) is preferably addition polymerisation. Suitable classes of monomers for step-growth polymerization are given in the group consisting of the melamine/ urea/ formaldehyde class, the isocyanate/ diol class (preferably the polyurethanes) and polyesters. Preferred are the melamine/urea formaldehyde class and the polyurethanes. Para 73 and 74 teach that the HPC deposition polymer is attached using vinyl acetate and or methyl acrylate.

Encapsulates (microcapsules) made using formaldehyde suffer from problems with evolution of formaldehyde and a formaldehyde scavenger is employed. This adds to chemical loading and can inhibit formulation flexibility. Lately, melamine formaldehyde, which has for many years been preferred over urea formaldehyde due to its reduced tendency to evolve formaldehyde, has come under pressure for elimination in consumer products due to its potential persistence in the environment. An alternative shell material for perfume microcapsules for delivery of other benefit agents in consumer products is therefore sought. Polyurethane has been proposed as an alternative but the reaction of isocyanates with polyols requires the use of further chemicals to initiate it and they can cause problems if they come into contact with many sensitive benefit agents. Typically a polyurethane capsule has to be finished off with an amine that reacts onto the large number of unreacted isocyanate sites in the polyurethane shell. This is a cumbersome procedure and makes these particles expensive to produce even if the initiators are compatible with the benefit agent contents. Addition of a nonionic polysaccharide deposition polymer to such a complex base particle would most likely require the use of addition polymers, yet further complicating the chemistry and adding cost.

It is desirable that the deposition polymer for an alternative shell material is the one already shown to be advantageous for melamine formaldehyde; i.e. a nonionic polysaccharide. To achieve the elimination of melamine formaldehyde from the product the attachment of such a deposition polymer to a non-melamine formaldehyde shell material cannot be achieved using the known pre-polymer route as commonly employed for melamine formaldehyde. The known alternative "addition polymer" route has never found favour outside of the laboratory because it complicates the manufacturing process excessively. It was therefore necessary to find a different shell wall material suitable for use with the advantageous nonionic deposition polymers and to devise a new process to attach those nonionic polysaccharide polymers to that shell wall material.

US 2012/0148644A1 (IFF) describes the synthesis of microcapsules with polyurea shells (para 180) for delivery of perfume as a benefit agent. The polyurea is preferentially formed using interfacial polymerisation of polyisocyanate and diamine. In the examples they disclose that carboxymethyl cellulose may be used to enhance perceived fragrance intensity (para 185). They also disclose adding deposition polymers (at start, during and after capsule formation) but these are cationic (or amphoteric) and not polysaccharide based (para 189). Merquat 100 is a highly changed cationic polymer.

US 2013/330292 (IFF) describes polyurea capsules, optionally containing a deposition aid. The capsules are said to be suited to consumer products. Charged and uncharged deposition aids are mentioned, optionally they can be attached via a covalent bond. Polysaccharides are mentioned as a possible deposition aid. In the preferred embodiments, where the capsules are included in consumer products such as hair conditioner, no deposition aid is used.

### SUMMARY OF THE INVENTION

According to the present invention there is provided a microcapsule containing composition comprising a surfactant or cationic material and at least 0.01 wt% of a microcapsule comprising a benefit agent inside a polyurea shell characterised in that the polyurea has a nonionic polysaccharide deposition polymer covalently bonded to it. Nonionic deposition polymers are known to provide enhanced deposition onto cellulosic materials for other types of shell material. When attached to polyurea we have surprisingly found that compared to when attached to prior art microcapsules they provide reduced agglomeration of the microcapsules and an ability for the microcapsules to remain better dispersed in surfactant containing products.

Preferably the deposition polymer is selected from the group consisting of mannan, glucan, glucomannan, xyloglucan, hydroxyalkyl cellulose, dextran, galactomannan and mixtures thereof. More preferably the deposition polymer is xyloglucan, galacto-manannan, dextran or hydroxypropyl cellulose and most preferably it is a xyloglucan or hydroxypropyl cellulose.

Preferred nonionic polysaccharides are those with a molecular weight Mw in excess of 40 kDa.

The weight ratio of deposition polymer to polyurea shell preferably lies in the range 1:500 to 1:2 as this achieves the surprising benefits previously noted. The inventive effect is advantageously obtainable at low levels of grafting of from 1:500 to 1:10, even 1:400 to 1:20, nonionic deposition polymer to polyurea shell.

The microcapsule preferably comprises at least 50 wt%, more preferably over 80 wt% core materials, including the benefit agent, and 1 to 30 wt% polyurea shell. More preferably the shell comprises up to 10 wt% of the microcapsule. The amount of shell is adjusted as known by the person skilled in the art to tune the friability of the microcapsule and to effectively retain its contents. The amount of benefit agent as a wt% of the total is preferably at least 10 wt%, more preferably at least 20 wt%, even more preferably at least 30 wt% and most preferably at least 40 wt% or even at least 50 wt%.

The deposition polymer level is preferably from 0.1 to 10 wt%, more preferably 0.2 to 5 wt%, based on microcapsule weight.

The polyurea may advantageously be formed by the reaction of poly-isocyanate and diamine. The poly functionality of the isocyanate is thought to provide sufficient free isocyanate reaction sites for the covalent bonding to occur easily.

Preferably, the microcapsule containing composition is a laundry detergent and the composition further comprises at least 5 wt% anionic surfactant.

Alternatively, the microcapsule containing composition is a skin cleansing composition or hair shampoo composition and the composition further comprises at least 2 wt% surfactant.

Alternatively, the microcapsule containing composition is a hair conditioner composition and the composition further comprises at least 1 wt% cationic material.

Compositions comprising the microcapsules are preferably consumer products and according to consumer preference and to mask the odour from other ingredients the compositions preferably further comprise at least 0.1 wt% perfume in addition to surfactant or cationic material.

Also according to the present invention there is provided a method of making the microcapsules, for use in the compositions of the invention, containing a benefit agent inside a polyurea shell which has a nonionic polysaccharide deposition polymer covalently bonded to it, wherein the nonionic polysaccharide is added at least 15 minutes after commencement of curing of the polyurea.

### DETAILED DESCRIPTION OF THE INVENTION

### The nonionic polysaccharide deposition polymer

Preferred nonionic polysaccharide deposition polymers may be selected from the group consisting of: tamarind gum (preferably consisting of xyloglucan polymers), guar gum, locust bean gum (preferably consisting of galactomannan polymers), and other industrial gums and polymers, which include, but are not limited to, Tara, Fenugreek, Aloe, Chia, Flaxseed, Psyllium seed, quince seed, xanthan, gellan, welan, rhamsan, dextran, curdlan, pullulan, scleroglucan, schizophyllan, chitin, hydroxyalkyl cellulose, arabinan (preferably from sugar beets), de- branched arabinan (preferably from sugar beets), arabinoxylan (preferably from rye and wheat flour), galactan (preferably from lupin and potatoes), pectic galactan (preferably from potatoes), galactomannan (preferably from carob, and including both low and high viscosities), glucomannan, lichenan (preferably from icelandic moss), mannan (preferably from ivory nuts), pachyman, rhamnogalacturonan, acacia gum, agar, alginates, carrageenan, chitosan, clavan, hyaluronic acid, heparin, inulin, cellodextrins, cellulose, cellulose derivatives and mixtures thereof.

Non-hydrolysable nonionic polysaccharides are most preferred. The polysaccharide preferably has a β-1,4-linked backbone. However, dextran which does not have such a backbone, is also preferred.

Preferably the polysaccharide is a cellulose, a cellulose derivative, or another β-1,4-linked polysaccharide having an affinity for cellulose, preferably mannan, glucan, glucomannan, xyloglucan, galactomannan and mixtures thereof. More preferably, the polysaccharide is selected from the group consisting of xyloglucan and hydroxypropyl cellulose. Galactomannan is typically from Locust bean gum and / or guar.

A highly preferred nonionic polysaccharide is Hydroxypropyl Cellulose with a molecular weight in excess of 40 kDa. Hydroxypropyl Cellulose (HPC) has the repeat structure shown in generalised terms below:

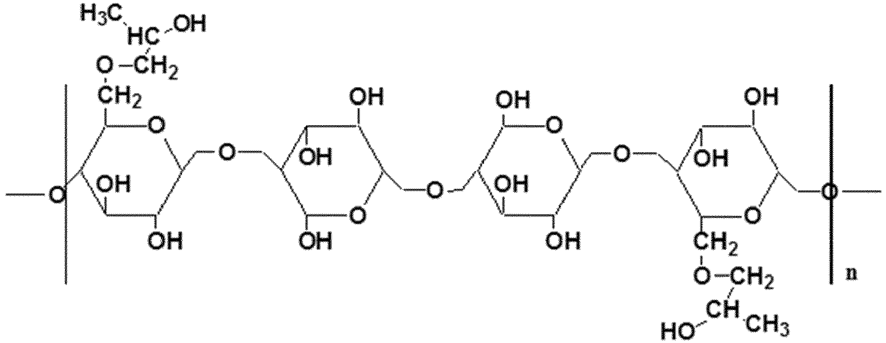

Especially good results may be obtained when the HPC is one with a viscosity in 2 wt% aqueous solution of 1000 to 4000 mPa.s. Viscosity measurements are done using a Brookfield viscometer, Spindle #3, @30 rpm. Lower viscosity materials are measured using Spindle #2, @60 rpm.

HPC is an ether of cellulose in which some of the hydroxyl groups in the repeating glucose units have been hydroxy-propylated forming -OCH₂CH(OH)CH₃ groups using propylene oxide. The average number of substituted hydroxyl groups per glucose unit is referred to as the degree of substitution (DS). Complete substitution would provide a DS of 3. However, as the hydroxy-propyl group itself contains a hydroxyl group, this can also be etherified during preparation of HPC. When this occurs, the number of moles of hydroxy-propyl groups per glucose ring, moles of substitution (MS), can be higher than 3.

The majority (typically around 75% for a DS of 3) of the mass of HPC is found in the substituent groups rather than the backbone.

Also, nonionic polysaccharides selected from the group consisting of: hydroxy-propyl methyl cellulose, hydroxy-ethyl methyl cellulose, hydroxy-propyl guar, hydroxy-ethyl ethyl cellulose and methyl cellulose may be used.

The ring spacing of these β-1,4-linked polymers is such that each alternate ring of the polymer is well placed to allow a pseudo hydrogen-bond interaction with the pi-electron clouds of the phthalate rings in polyester. Moreover, these polymers have a balance of hydrophobicity and hydrophillicity which means that they are able to interact with a fabric without being so hydrophobic as to be insoluble. Other nonionic, modified polysaccharides, for example hydroxyl-ethyl cellulose, do not have the correct properties and show poor performance as deposition polymers, especially on polyester.

In those ethers of cellulosics in which some of the hydroxyl groups in the repeating glucose units have been hydroxy-alkylated the average number of substituted hydroxyl groups per glucose unit is referred to as the degree of substitution (DS). Complete substitution would provide a DS of 3. However, if the substituent group itself contains a hydroxyl group, this can also be etherified. When this occurs, the number of moles of substituent groups per glucose ring, moles of substitution (MS), can be higher than 3.

Some of the -OH groups (where present) in the hydroxyl-alkyl pendant group may be replaced with alkyl ethers. Typically these are C₁-C₂₀ alkyl ethers, and may, in specific cases be C₁₆-C₂₂ ethers. The most preferred alkyl chain is stearyl.

Hydroxy-propyl methyl cellulose (HPMC), has the repeat structure shown in generalised terms below:

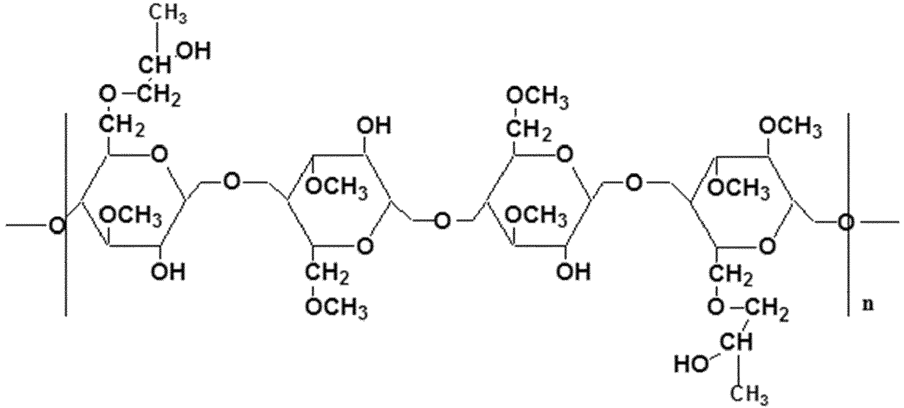

Since the hydroxypropoxy substituents can be attached to each other on side chains, the degree of substitution for HPMC can be higher than 3.

In useful derivatives of HPMC "Sangelose" some of the -OH groups in the hydroxylpropyl pendant group are replaced with alkyl ethers. Typically these are C₁-C₂₀ alkyl ethers, and may, in specific cases be C₁₆-C₂₂ ethers. The most preferred alkyl chain is stearyl.

Hydroxy-ethyl methyl cellulose (HEMC), has the repeat structure shown in generalised terms below:

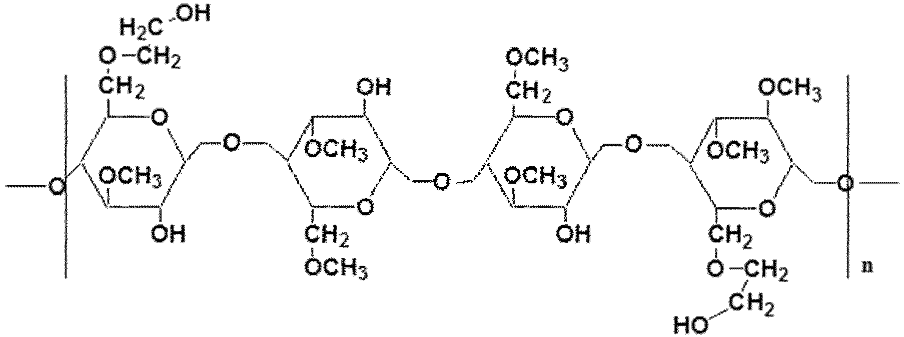

Since the ethoxy substituents can be attached to each other on side chains, the degree of substitution can be higher than 3.

Hydroxy-propyl guar (HPG), has the repeat structure shown in generalised terms below:

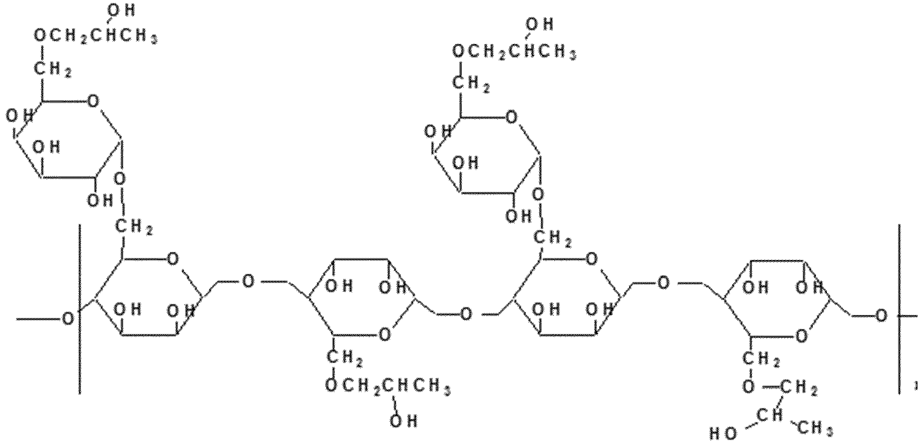

Since the hydroxypropoxy substituents can be attached to each other on side chains, the degree of substitution in HPG can be higher than 3.

Hydroxy-ethyl ethyl cellulose (HEEC), has the repeat structure shown in generalised terms below:

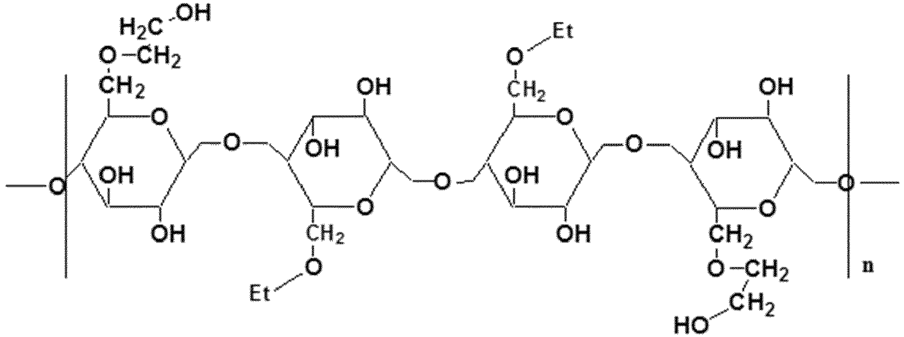

HEEC is less preferred than other nonionic polysaccharide delivery aids disclosed herein.

Methyl cellulose (ME), has the repeat structure shown in generalised terms below:

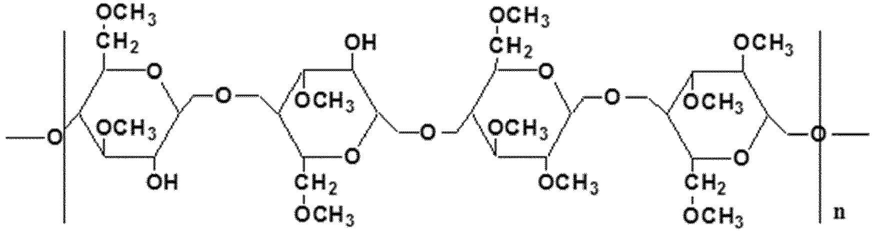

The theoretical maximum degree of substitution (DS) is 3.0. However, more typical values are 1.3 to 2.6.

Especially good results may be obtained when the deposition polymer is one which has a viscosity in 2 wt% aqueous solution of over 1000 mPa.s. Viscosity measurements are made using a Brookfield viscometer, Spindle #3, @30 rpm. Lower viscosity materials are measured using Spindle #2, @60 rpm.

Preferably the nonionic polysaccharide deposition polymer has a molecular weight above 50 kDa and more preferably above 140 kDa, most preferably above 500 kDa. As the molecular weight is increased the performance of the deposition polymer generally increases.

DS is typically in the range from 1.0 to 3, more preferably above 1.5 to 3, most preferably, where possible from 2.0 to 3.0.

A typical MS for the deposition polymer is 1.5 to 6.5. Preferably the MS is in the range from 2.8 to 4.0, more preferably above 3.0, most preferably from 3.2 to 3.8.

Preferably, the deposition-aid polymer is present at levels such that the ratio, polymer: particle solids, is in the range 1:500 to 3:1, more preferably 1:500 to 1:2 and most, preferably 1:200 to 1:2.

### The Polyurea

Polyureas are formed from diisocyanates or polyisocyanates with diamines or polyamines. Normally to make a microcapsule the cyanate part is present in a dispersed (oily) phase and the amine part is present in the continuous (aqueous) phase. The shell forms via interfacial polymerisation at the phase interface. This reaction and the manufacture of polyurea shell microcapsules is widely known and understood to the skilled person and the invention can be applied to any type of polyurea microcapsule.

When a diisocyanate is used it may be linear aliphatic, cycloaliphatic or aromatic.

Suitable, aromatic polyisocyanates comprise, but are not limited to, 2,4-and 2,6-toluene diisocyanate, naphthalene diisocyanate, diphenyl methane diisocyanate and triphenyl methane-p,p'p"- trityl triisocyanate, polymethylene polyphenylene isocyanate, 2,4,4'-diphenylether triisocyanate, 3,3'-dimethyl-4,4'- diphenyl diisocyanate, 3,3'-dimethoxy-4,4'diphenyl diisocyanate, and 4,4'4"-triphenylmethane triisocyanate.

Suitable aliphatic polyisocyanates comprise, but are not limited to dicyclohexylmethane 4,4 '-diisocyanate, hexamethylene-1,6-diisocyanate, isophorone diisocyanate, trimethylhexamethylene diisocyanate, trimer of hexamethylenel,6-diisocyanate, trimer of isophorone diisocyanate, 1 ,4-cyclohexane diisocyanate, urea of hexamethylene diisocyanate, trimethylene diisocyanate, propylene-1 ,2-diisocyanate and butylenes-1,2-diisocyanate and mixtures thereof.

Suitable diamines can comprise amines such as ethylene diamine (EDA), phenylene diamine, toluene diamine, hexamethylene diamine, diethylenetriamine, tetraethylene pentaamine, pentamethylene hexamine, 1,6-hexane diamine, Methylene tetramine, 2,4-diamino-6-methyl- 1,3,5 triazine 1,2-diaminocyclohexane, 4,4'-diamino-diphenylmethane, 1,5-diaminonaphthalene, 2,4,4'- triaminodiphenylether, bis(hexa-methylenetriamine), 1,4,5,8-tetraaminoanthraquinone, isophorone diamine, diamino propane and diaminobutane, and mixtures thereof. Polymeric amines may also be used, for example Jeffamines (polyether amine) and poly(ethyleneimine).

Water soluble diamine or amine salt or polyamines or polyamines salts are preferred as the amine is usually present in the aqueous phase. Combinations of Polyisocyanates and diamines are preferred because the plural functionality enables networked structures and having the plural functional material in the disperse phase reduces the chance of bonds forming between adjacent particles and clumping resulting

Other suitable materials are disclosed, for example, in US2014/0017287. Isocyanate-based capsule wall technologies are also disclosed in WO 2004/054362; EP 0 148149; EP 0 017 409; U.S. 4,417,916, U.S. 4,124,526, US 6,566,306, US 6,730,635, WO 90/08468, WO 92/13450, US 4,681, 806, US 4,285,720,US 6,340,653 and EP 2673078.

### The benefit agent

A benefit agent is a material that when deposited onto a substrate imparts some desirable effect to that substrate. As such it comprises a wide selection of materials. Because polyurea capsules are made by interfacial polymerisation from an oil in water emulsion the benefit agent is generally a hydrophobic material that is miscible with an oil phase or forms the oil phase.

Suitable benefit agents include perfume raw materials (also referred to herein as fragrance), silicone oils, waxes, hydrocarbons, higher fatty acids, essential oils, lipids, skin coolants, vitamins, sunscreens, antioxidants, malodour reducing agents, odour controlling materials, skin softening agents, insect and moth repelling agents, colorants, chelants, sanitization agents, germ control agents , skin care agents, natural actives, antibacterial actives, preservatives, chemosensates, (for example menthol), sunless-tanning agents (for example dihydroxyacetone), emollients (for example sunflower oil and petrolatum), antiaging agents, anti-inflammatory agents, skin conditioning agents, skin lightening agents and mixtures thereof.

Preferred benefit agents are fragrance, anti-aging agents, antimicrobial agents, anti-bacterial agents, anti-fungal agents, lubricants and shading dyes. Other possible benefit agents include: anti-oxidants, vitamins, anti-inflammatory actives, skin lightening agents, skin conditioning agents, for example 12-hydroxy stearic acid, oils, insect repellents and sunscreens. These are described in detail in the literature. For example: WO13/026657 and WO12/022736.

### Optional materials in the microcapsule (including wall)

Isocyanate must be soluble in whatever will form the microcapsule core so additional material may be needed if the benefit agent does not dissolve the isocyanate.

The microcapsule may therefore optionally comprise a carrier oil (also referred to herein as a diluent). It will be clear to a skilled person which oils are suitable for use with a certain benefit composition. The carrier oils are hydrophobic materials that are miscible in the benefit agent materials used in the present invention. Suitable oils are those having reasonable affinity for the benefit agent. Suitable materials include, but are not limited to triglyceride oil, mono and diglycerides, mineral oil, silicone oil, diethyl phthalate, polyalpha olefins, castor oil and isopropyl myristate. Preferably, the oil is a triglyceride oil, most preferably a capric/caprylic triglyceride oil.

Many emulsifying agents are known for use in emulsion polymerisation. Suitable emulsifying agents for use in the polymerisation process may comprise, but are not limited to, nonionic surfactants such as polyvinylpyrrolidone (PVP), polyethylene glycol sorbitan monolaurate (Tween 20), polyethylene glycol sorbitan monopalmitate (Tween 40), polyethylene glycol sorbitan monooleate (Tween 80), polyvinyl alcohol (PVA), and poly(ethoxy)nonyl phenol, ethylene maleic anhydride (EMA) copolymer, Easy-Sperse™ (from ISP Technologies Inc.), ionic surfactants such as partially neutralized salts of polyacrylic acids such as sodium or potassium polyacrylate or sodium or potassium polymethacrylate. Brij™-35, Hypermer™ A 60, or sodium lignosulphate, and mixtures thereof.

Emulsifiers may also include, but are not limited to, acrylic acid-alkyl acrylate copolymer, poly(acrylic acid), polyoxyalkylene sorbitan fatty esters, polyalkylene co-carboxy anhydrides, polyalkylene co-maleic anhydrides, poly(methyl vinyl ether-co-maleic anhydride), poly(propylene-co-maleic anhydride), poly(butadiene co-maleic anhydride), and poly(vinyl acetate-co-maleic anhydride), polyvinyl alcohols, polyalkylene glycols, polyoxyalkylene glycols, and mixtures thereof.

Preferred emulsifying agents are fatty alcohol ethoxylates (particularly of the Brij™ class), salts of ether sulphates (including SLES), alkyl and alkaryl sulphonates and sulphates (including LAS and SDS) and cationic quaternary salts (including CTAC and CTAB).

Typically a co-surfactant will be present in the dispersed phase and in the resulting particle. Suitable co-surfactants for use in the present invention include hexadecane, cetyl alcohol, lauroyl peroxide, n-dodecyl mercaptan, dodecyl methacrylate, stearyl methacrylate, polystyrene, polydecene, mineral oils, isopropyl myristate, C₁₂-C₁₅ alkyl benzoate and polymethyl methacrylate.

The preferred cosurfactants comprise hexadecane, polydecene and isopropyl myristate.

As a wt% of oil phase as a total, the co-surfactant is typically 0 to 20 wt%, preferably 1 to 15 wt%, more preferably 2 to 12.5 wt%.

In the prior art it has been suggested that pure polyurea shell walls are not useful due to their inherent leakiness. Inclusion of materials such as Carboxymethyl cellulose into the wall is taught as are more complex shell walls made of mixtures of polyurea and other polymers. In the present invention it is advantageous for the capsule wall to be at least 95% and preferably substantially 100% polyurea. Capsule leakiness can be adjusted by wall thickness and choice of the amine(s) and isocyanate(s). For some applications, e.g. laundry, it is desirable that some benefit agent leaks from the capsule before it ruptures. When the benefit agent is a perfume this can increase freshness perception when the washing is still wet.

### Size of microcapsule

Generally, the microcapsules will have an average diameter of from about 0.001 to about 1,000 microns, preferably from about 1 to about 500 microns, more preferably from about 5 to about 100 microns, and even more preferably from about 10 to about 50 microns. These dimensions can play an important role in the ability to control the application of the microcapsule composition in the practice of the present invention. The broadest range of microcapsule size under any conditions would be about 0.001 to about 1,000 microns and a more easily sprayed size limit would be between about 10 and about 50 microns.

It is desirable for a suspension of microcapsules to contain a dispersant also in order to prevent microbial contamination it is desirable that the microcapsule composition contains a preservative. The preservative may be contained in the core material and/or in the aqueous carrier.

### Uses of the microcapsules and suitable compositions

The compositions for use in the invention may contain one or more other ingredients in addition to the polyurea microcapsules with nonionic polysaccharide deposition polymer. They may contain other types of microcapsules or similar microcapsules with a different benefit agent inside.

In addition to any fragrance formulation that may be contained within the microcapsules, a composition comprising microcapsules of the present invention may also contain free perfume.

Formulated compositions comprising the particles of the invention may contain a surface-active compound (surfactant) which may be chosen from soap and non-soap anionic, cationic, nonionic, amphoteric and zwitterionic surface active compounds and mixtures thereof. Many suitable surface active compounds are available and are fully described in the literature, for example, in "Surface-Active Agents and Detergents", Volumes I and II, by Schwartz, Perry and Berch. The preferred surface-active compounds that can be used are soaps and synthetic non soap anionic, and nonionic compounds.
Depending on the end use of the composition comprising the microcapsules other materials commonly found in such compositions may be used as usual. The one main exception is that free cellulase is preferably absent from the composition unless it is somehow deactivated. Mannanase is also desirably avoided.

Such ingredients include preservatives (e.g. bactericides), pH buffering agents, perfume carriers, anti-redeposition agents, soil-release agents, polyelectrolytes, anti-shrinking agents, anti-wrinkle agents, anti-oxidants, sunscreens, anti-corrosion agents, drape imparting agents, anti-static agents, pearlisers and/or opacifiers, natural oils/extracts, processing aids, e.g. electrolytes, hygiene agents, e.g. anti-bacterials and antifungals, thickeners, skin benefit agents, colorants, whiteners, optical brighteners, soil suspending agents, detersive enzymes, compatible bleaching agents (particularly peroxide compounds and active chlorine releasing compounds), gel-control agents, freeze-thaw stabilisers, bactericides, preservatives (for example 1,2-benzisothiazolin-3-one), hydrotropes, perfumes and mixtures thereof.

The compositions for use in the invention may also contain pH modifiers such as hydrochloric acid or lactic acid.

The end-product compositions of the invention may be in any suitable physical form e.g. a solid such as a solid bar, a paste; or a liquid or gel, preferably, an aqueous-based liquid.

Laundry detergents, skin cleansing compositions and hair conditioners are preferred compositions for use with the microcapsules.

The microcapsules are typically included in compositions at levels of from 0.01% to 10%, preferably from 0.5% to 7%, most preferably from 0.5% to 5% by weight of the total composition.

The invention will now be further described with reference to the following non-limiting examples.

### EXAMPLES

### Example 1 - Synthesis of Polyurea Microcapsule with Xyloglucan Added at Various Addition Times

A number of polyurea microcapsules were synthesised, whereby the xyloglucan deposition polymer was added over various addition times, once the encapsulating process had begun. The materials sued are given in Table 1 and composition details are given in Table 2.

**Table 1**

| **Material** | **Supplier** | **% Active** |
|---|---|---|
| Benzyl Acetate | Aldrich | >99% |
| Hostasol Yellow 3G | Clariant | 100% |
| Poly(methylene(polyphenyl)isocyanate) | Polysciences | 100% Active (30% Isocyanate) |
| LAS (Linear Alkyl Benzene Sulphonate, Sodium Salt) | Petrelabs | 90% |
| Hexamethylene Diamine | Aldrich | 98% |
| Xyloglucan | DSP Gokyo Food and Chem. Co. | 100% |

**Table 2**

| | |
|---|---|
| Particle Solids (%) = | 15 |
| % BA (on solids) = | 89.6 |
| % Hostasol (on oil phase)= | 0.02 |
| % PMPHI (on solids) = | 7.2 |
| %HMDA (on solids) = | 3.2 |
| Total Wt (g) = | 200 |
| %XG (on solids) = | 2.0 |

The microcapsules were made using the following synthesis:
1) 0.0054 g of Hostasol yellow 3G and 2.2 g of poly(methylene(polyphenyl)isocyanate) were dissolved in 26.9 g of benzyl acetate (oil phase).
2) 0.77 g of LAS (linear alkyl benzene sulfonate) was dissolved in 100 ml of deionised water by stirring for 30 minutes (aqueous phase).
3) A 1 wt% xyloglucan (XG) solution was prepared separately by homogenising (8,000 rpm) 5 g XG in 495 g of boiled distilled water for 5 minutes and allowing to cool before use.
4) The oil and aqueous phases were mixed and emulsified using a homogeniser (5,000 rpm) for 2 minutes (care was taken to avoid excessive foaming).
5) This mixture was constantly stirred using an overhead stirrer (200 rpm).
6) The water for solids adjustment was added (64.2 g for XG free sample and 4.2 g for all XG containing samples).
7) Then 0.96 g hexamethylene diamine dissolved in 5 g of deionised water was added dropwise.
8) Numerous samples were prepared, one where no XG solution was added (control) and the others whereby 60 g of the 1wt% XG solution was added after 7, 15, 30, 45 or 60 minutes.
9) Polyurea curing was allowed to proceed at room temperature for at least 3 hours.

### Example 2 - Deposition Assessment via Fluorescence Depletion

### 1) Preparation of Wash Liquor Stock Solutions

A concentrated wash liquor stock solution (10X) was prepared using the materials in Table 3.

**Table 3**

| **Component** | **Active (%)** | **Amount (g)** |
|---|---|---|
| LAS (Linear Alkyl Benzene Sulphonate, Sodium Salt) | 90.0% | 5.5 |
| Neodol 25-7 | 100.0% | 5.0 |
| Na2CO₃ | 100.0% | 7.55 |
| NaHCO₃ | 100.0% | 2.42 |
| Deionised water | N/A | 979.5 |

### 2) Preparation of the wash liquor:

The wash liquor stock (10 ml) and deinonised water (90 ml) were added to a 125 ml plastic bottle. This gave 100 ml of wash liquor buffered to pH 10.5 and containing 1 g/L surfactant (50:50 LAS:Neodol 25-7).

### 3) Simulated Wash:

0.005 g (50 ppm on wash liquor) of microcapsule particles were added to the plastic bottle containing wash liquor and agitated slightly to ensure mixing. (Washes were done in duplicate for each sample and results averaged). A 5 ml aliquot was taken from each and the fluorescence (excitation = 450 nm, emission = 515, slit width = 15) measured in a 1 cm cuvette using a fluorimeter (Perkin Elmer LS50). This fluorescence value represents 100% particles in the wash solution prior to the simulated wash process.

A section of unfluoresced cotton measuring 20 cm by 20 cm was placed into each plastic bottle containing the wash liquor and microcapsules and the bottle sealed.

The bottles were agitated at 40 °C and 150 rpm using a water shaker bath (Haake SWB25) for 45 minutes to simulate the main wash.

The cloths were then removed and wrung by hand and a 5 ml aliquot of the remaining wash liquor was taken and the fluorescence measured (as before). From interpolation of a concentration versus florescence calibration curve, the concentration of the microcapsules remaining in the liquor after the wash could be determined and hence the percentage deposited (wash deposition) on cloth determined by difference.

The plastic bottles were then thoroughly rinsed and the 'wrung' cloths returned to the bottles and 100 ml of de-ionised water was added and the bottles sealed. The bottles were then agitated (150 rpm) on the water shaker bath for 10 minutes at ambient temperature (∼20°C) to simulate a rinse procedure. The clothes were then removed and wrung by hand. A 5 ml aliquot of the rinse solution was taken and the fluorescence determined as before. Again, interpolation of the calibration plot allowed the microcapsule concentration removed from the cloth during the rinse to be determined and, by subtraction from the level deposited after the main wash, the overall percentage deposition was determined.

The percentage depositions (after main wash and rinse) for the samples prepared in Example 1) are shown in Table 4.

**Table 4**

| | Main Wash | | After Rinse | |
|---|---|---|---|---|
| Polyurea - XG Addition Time | Depo (%) | SD | Depo (%) | SD |
| Control (No XG) | 58.4 | 5.0 | 44.5 | 5.6 |
| After 7 mins | 69.9 | 5.3 | 46.4 | 13.2 |
| After 15 mins | 83.1 | 3.0 | 72.6 | 0.1 |
| After 30 mins | 79.9 | 1.9 | 69.5 | 3.1 |
| After 45 mins | 86.3 | 4.4 | 73.3 | 1.2 |
| After 60 mins | 85.3 | 1.1 | 69.0 | 2.8 |

The results show that deposition is enhanced by the attachment of xyloglucan and that for maximum deposition, the xyloglucan should not be added until 15 minutes into the reaction. After this time, addition up to one hour is possible without detriment to deposition performance.

### Example 3 - Synthesis of Comparative Melamine Formaldehyde Microcapsule

A comparative example prepared at equivalent composition levels and a xyloglucan addition time point of 15 minutes into microcapsule formation was prepared, with the difference that melamine formaldehyde was used as shell material (rather than polyurea) and the reaction was carried out at 75°C for 3 hours. The composition details are given in Table 5 and the materials used in Table 6.

**Table 5**

| | |
|---|---|
| Particle Solids (%) | 15.0 |
| Benzyl Acetate (% on solids) | 89.6 |
| Hostasol (% on oil phase) | 0.02 |
| Melamine Formaldehyde (% on solids) | 10.4 |
| Xyloglucan (% on solids) | 2.0 |
| Total Weight (g) | 200 |

**Table 6**

| **Material** | **Supplier** | **% Active** |
|---|---|---|
| Melamine | Aldrich | 100% |
| Formaldehyde Solution (Formalin) | Aldrich | 37.0% |
| Benzyl Acetate | Aldrich | >99% |
| Sodium Chloride | Aldrich | >99% |
| Xyloglucan | DSP Gokyo Food and Chem. Co. | 100% |
| Formic Acid | Aldrich | >96% |
| Sodium Carbonate | Aldrich | >99% |

### Synthesis:

### Melamine Formaldehyde Pre-Polymer Synthesis

1) To a 250 ml reaction flask was added 19.5 g formalin (37 wt% aqueous formaldehyde) and 44 g water.
2) The pH of the solution was adjusted to 8.9 using 5 wt% sodium carbonate aqueous solution.
3) 10 g melamine and 0.6 g sodium chloride were added and the mixture stirred at 200 rpm using an overhead stirrer.
4) The mixture was heated to 62 °C and stirring continued until the mixture became clear (typically 1 hour). This solution contains 23.2 wt% trimethylol melamine and is hereinafter referred to as pre-polymer (1).

### Xyloglucan Grafted Melamine Formaldehyde Microcapsule Synthesis

A number of xyloglucan grafted microcapsules were prepared containing 2, 1 or 0.5% xyloglucan (on particle weight). The weights used are given Table 7.

**Table 7**

| Material: | 2% XG | 1% XG | 0.5% XG |
|---|---|---|---|
| Pre-Polymer (1) | 13.4 | 13.4 | 13.4 |
| Benzyl Acetate | 26.9 | 26.9 | 26.9 |
| Hostasol Yellow 3G | 0.0054 | 0.0054 | 0.0054 |
| Water | 99.7 | 129.7 | 144.7 |
| 1% Xyloglucan Solution | 60.0 | 30.0 | 15.0 |

The xyloglucan solution was prepared by dissolving 2 g of xyloglucan in 198 g of boiled water, using a homogeniser at 8,000 rpm for 5 minutes and allowing to cool prior to using.
1) The water was added to a 250 ml reaction flask and heated to 75°C.
2) The pre-polymer (1) was then added, and the pH was adjusted to 4.1 using 10 wt% formic acid.
3) After approximately 1 minute the solution became turbid.
4) A homogeniser (at 8,000 rpm) was then used to agitate the mixture while slowly adding the benzyl acetate over ∼30seconds.
5) The mixture was agitated at this shear regime for a further 2 minutes.
6) The reaction vessel was then sealed and heated with overhead stirring (300 rpm) at 75°C.
7) The xyloglucan solution was added after 15 minutes.
8) The reaction was allowed to continue for a total of 3 hours.
9) The mixture was cooled and adjusted to pH 7 using 5 wt% sodium carbonate

The final dispersions were 15% particle solids, consisting of approximately 10% MF shell and 90% Benzyl Acetate, with 2 to 0.5% XG (on particle solids) attached.

### Example 4 - Comparison of Particle Size of Xyloglucan Grafted Polyurea and Melamine Formaldehyde Microcapsules

The particle size for both the polyurea (PU) and melamine formaldehyde (MF) synthesized microcapsules was measured (using a Malvern Mastersizer 2000). The results are given in Table 8.

**Table 8**

| | Particle Size | d(0.5) microns |
|---|---|---|
| **XG Level (%)** | **PU** | **MF** |
| 2 | 15.6 | 139.1 |
| 1 | 14.9 | 20.7 |
| 0.5 | 14.9 | 17.3 |

The PU synthesis route generates smaller particles and allows the incorporation of higher XG levels without inducing aggregation and increasing particle size.

### Example 5 - Comparison of Laundry Liquid Stability of Xyloglucan Grafted Polyurea and Melamine Formaldehyde Microcapsules

The stability of the PU and MF microcapsules as used in Example 4 were compared within an unstructured laundry liquid detergent with composition in Table 9 (1 wt% of microcapsules added).

**Table 9**

| Component | % w/w |
|---|---|
| Monopropylene Glycol | 11 |
| Glycerol | 5 |
| Monoethanolamine | 7 |
| Triethanolamine | 2.5 |
| Citric Acid | 3.9 |
| Neodol 25-7 | 4.6 |
| LAS Acid | 8.8 |
| Prifac 5908 | 3 |
| Dequest 2010 | 1.5 |
| SLES 3EO | 6.8 |
| EPEI (Sokalan HP 20) | 3 |
| Acusol OP301 | 0.1 |
| Sodium Sulphite | 0.25 |
| Water | to 100% |

Attachment of a nonionic polysaccharide to a microcapsule might be expected to slightly stabilize a dispersion of the particles. Surprisingly the same deposition polymer when added to polyurea microcapsules gave a visibly much more stable suspension of the particles after 3 days, whereas the corresponding melamine formaldehyde capsules with the same level of the same deposition polymer attached visibly settled out of the detergent liquid much more over the same time period.

### Example 6 - Synthesis of Full Perfume Polyurea Microcapsule with Grafted Hydroxypropyl Cellulose

This example details the production of hydroxypropyl cellulose (HPC) grafted polyurea encapsulates containing a model perfume (rather than benzyl acetate only) and low levels of hydroxypropyl cellulose (0.3% on encapsulate weight). The materials used are similar to those in Example 1, with the exceptions that AKK perfume replaces benzyl acetate, 5% polyvinyl alcohol aqueous solution replaces 0.5% LAS solution and 1% hydroxypropyl cellulose aqueous solution replaces 1% XG solution. New material details are given in Table 1 and composition details are given in Table 2.

**Table 10**

| **Material** | **Supplier** | **% Active** |
|---|---|---|
| AKK Perfume | Aldrich (for ingredients) - See Table 11 for composition | 100% |
| Polyvinyl Alcohol (Mowiol 18-88) | Aldrich | 100% |
| Hydroxypropyl Cellulose (MW = 370K) | Aldrich | 100% |

**Table 11 (AKK Perfume Composition)**

| **Wt%** | **Ingredient** | **CAS** |
|---|---|---|
| 6.66 | Manzanate | 39255-32-8 |
| 6.66 | Aldehyde C8 | 124-13-0 |
| 6.66 | Tetra hydro linalol | 78-69-3 |
| 6.66 | Benzyl acetate | 140-11-4 |
| 6.66 | Linalyl acetate | 115-95-7 |
| 11.7 | OTBCA | 88-41-5 |
| 1.66 | Damascone, delta | 57378-68-4 |
| 6.66 | Aldehyde c12 | 112-54-9 |
| 6.66 | Verdyl acetate | 5413-60-5 |
| 6.66 | Ionone beta | 14901-07-6 |
| 6.66 | Bangalol | 28219-61-6 |
| 6.66 | Iso E super (OTNE) | 54464-57-2 |
| 6.66 | Hexyl cinnamic aldehyde | 101-86-0 |
| 6.66 | Cyclopentadecanolide | 106-02-5 |
| 6.66 | Phenyl ethyl 2-phenylacetate | 102-20-5 |

The weights and procedure were identical to that given in Example 1. The HPC solution was added 15 minutes after the reaction started.

The final dispersion was 10% particle solids, consisting of approximately 10% PU shell and 90% AKK Perfume, with 0.3% HPC (on particle solids) attached.

### Example 7 - Synthesis of Comparative Full Perfume Melamine Formaldehyde Encapsulate with Grafted Hydroxypropyl Cellulose

A comparative example prepared at equivalent composition levels and an HPC addition time point of 15 minutes into microcapsule formation was prepared. This comparative example utilises MF shell rather than polyurea. The materials used were similar to those detailed in Example 3, with the exceptions that AKK perfume replaces benzyl acetate and 1% hydroxypropyl cellulose aqueous solution replaces 1% XG solution. New material details are given in Tables 10 and 11.

The weights and procedure were identical to that detailed in Example 3.

The final dispersion was 10% particle solids, consisting of approximately 10% MF shell and 90% AKK Perfume, with 0.3% HPC (on particle solids) attached.

### Example 8 - Comparison of Laundry Liquid Stability of HPC Grafted Polyurea and Melamine Formaldehyde Microcapsules

The stability of the PU and MF microcapsules as described in Examples 6 and 7 were compared within an unstructured laundry liquid detergent. The inclusion levels and procedure were identical to that detailed in Example 5.

A clear difference in stability was observed after three days:
The MF encapsulate had completely phase separated and creamed to the top of the solution, whereas with the polyurea variant the stability is maintained.

### Examples 9 to 15: Compositions

These examples show the use of the PU microcapsules in typical laundry, hair and skin body wash compositions. The materials used are given in Table 12.

**Table 12**

| **Component** | **Specification** |
|---|---|
| LAS | Linear Alkyl Benzene Sulphonic Acid |
| | Marlon AS3, ex. Huls |
| Na-PAS | Primary Alkyl Benzene Sulphonic Acid, neutralises with NaOH |
| Dobanol 25-7 | C12-15 Ethoxylated Alcohol, 7EO, ex Shell |
| Zeolite | Wassalith P, ex Degussa |
| STPP | Sodium Tri Polyphosphate, Thermphos NW, ex Hoechst |
| Dequest 2066 | Metal Chelating Agent, ex. Monsanto |
| Lipolase | Type 100L, ex. Novo |
| Savinase 16L | Protease, ex Novo |
| Sokalan CP5 | Acrylic/Maleic Builder Polymer, ex BASF |
| Deflocculating Polymer | Polymer A-11 disclosed in EP-A-346 995 |
| SCMC | Sodium Carboxymethyl Cellulose |
| Texapon N 701 | Sodium Laureth Sulphate, ex Cognis |
| Aculyn 28 Polymer (20%) | Carbomer, ex Rohm and Haas |
| Tegobetaine CK | Cocoamidopropyl Betaine, ex Goldschmidt |
| BF Jaguar C14 | Guar Hydroxypropyltrimonium Chloride, ex Aqualon |
| DC 1788 | Dimethiconol (and) TEA-Dodecylbenzenesulfonate, ex Dow Corning |
| Euperlan KE 4515 | Glycol Distearate and Sodium Laureth Sulphate , Cocamidopropyl Betaine, ex Cognis |
| BTAC | Genamin BTLF, Behentrimonium Chloride, ex Aako |
| Cetearyl Alcohol | Lanette S3, ex. BASF |
| DC5 7134 | Silicone Emulsion, ex Dow Corning |
| Carbopol Aqua SF-1 | Acrylate Copolymer, Lubrizol |
| SLES 1EO | Sodium Lauryl Ether Sulphate (1EO), ex. BASF |
| Cocamide MEA | Cocamide monoethanolamine, ex Stepan |
| Versene XL100 | Ethylenediaminetetraacetic Acid Tetrasodium Salt, ex Dow Chemical |
| PPG-9 | Polypropylene Glycol, MW = 425g, Dow Chemical |
| Laundry Minors | Anti-redeposition polymers, transition-metal scavengers, bleach stabilisers, fluorescers, antifoam, dye transfer inhibition polymers, enzymes, perfume |

### Example 9 - Spray-Dried Powder

Table 13 gives the composition of a spray dried laundry detergent powder containing PU microcapsules from Example 1.

**Table 13**

| **Component** | **% w/w** |
|---|---|
| PU Microcapsules (from Example 1) | 1.0 |
| Na PAS | 11.5 |
| Dobanol 25-7 | 6.3 |
| Soap | 2.0 |
| Zeolite | 24.1 |
| SCMC | 0.6 |
| Na Citrate | 10.6 |
| Na Carbonate | 23.0 |
| Sodium Polyacrylate (MW 1,300,000) | 0.7 |
| Dequest 2066 | 0.4 |
| Sokalan CP5 | 0.9 |
| Savinase 16L | 0.7 |
| Lipolase | 0.1 |
| Perfume | 0.4 |
| Water/salts | Up to 100% |

### Example 10 - Laundry Detergent Granulate Prepared by Non-Spray Drying Method

Table 14 gives a granular laundry detergent composition containing the PU microcapsules of Example 1, as can be manufactured by a non-tower route.

**Table 14**

| **Component** | **% w/w** |
|---|---|
| PU Microcapsules (from Example 1) | 1.0 |
| Na PAS | 13.5 |
| Dobanol 25-7 | 2.5 |
| STPP | 45.3 |
| Na Carbonate | 4.0 |
| Polyacrylamide (MW 5,500,000) | 2.8 |
| Na Silicate | 10.1 |
| Perfume | 1.0 |
| Minors | 1.5 |
| Water | Up to 100% |

### Example 11 - Isotropic Laundry Liquid

Table 15 gives an isotropic laundry liquid composition incorporating the PU microcapsules of Example 1.

**Table 15**

| **Component** | **% w/w** |
|---|---|
| PU Microcapsules (from Example 1) | 1.0 |
| Na Citrate | 10.7 |
| Propylene Glycol | 7.5 |
| Ethylene Glycol | 4.5 |
| Borax | 3 |
| Savinase 16L | 0.3 |
| Lipolase | 0.1 |
| Dextran (MW 800,000) | 1.0 |
| Monoethanolamine | 0.5 |
| Cocofatty Acid | 1.7 |
| NaOH (50%) | 2.2 |
| LAS | 10.3 |
| Dobanol 25-7 | 6.3 |
| LES | 7.6 |
| Perfume | 1.0 |
| Minors (adjust pH to 7 with NaOH) | 1.3 |
| Water | Up to 100% |

### Example 12 - Structured Laundry Liquid

Table 16 gives a structured liquid laundry detergent composition incorporating the PU microcapsules of Example 1.

**Table 16**

| **Component** | **% w/w** |
|---|---|
| PU Microcapsules (from Example 1) | 1.0 |
| LAS | 16.5 |
| Dobanol 25-7 | 9.0 |
| Oleic Acid (Priolene 6907) | 4.5 |
| Zeolite | 15.0 |
| KOH (neutralising acids to pH 7) | 8.5 |
| Citric Acid | 8.2 |
| Deflocculating Polymer | 1.0 |
| Protease | 0.38 |
| Lipolase | 0.2 |
| Imacol C (ex Clariant) | 2.0 |
| Perfume | 1.0 |
| Minors | 0.4 |
| Water | Up to 100% |

### Example 13 - Hair Shampoo

Table 17 gives a hair shampoo composition incorporating the PU microcapsules of Example 1.

**Table 17**

| **Composition** | **% w/w** |
|---|---|
| PU Microcapsules (from Example 1) | 1.0 |
| Texapon N701 | 12.8 |
| Aculyn 28 Polymer (20%) | 1.0 |
| Tegobetaine CK | 1.6 |
| BF Jaguar C14 | 0.15 |
| DC 1788 | 3.0 |
| Emperlan KE 4515 | 2.5 |
| Propylene Glycol | 0.25 |
| Sodium Hydroxide | 0.2 |
| Perfume | 2.0 |
| Water | Up to 100% |

### Example 14 - Hair Conditioner

Table 16 gives a hair conditioner composition incorporating the PU microcapsules of Example 1.

**Table 16**

| **Composition** | **% w/w** |
|---|---|
| PU Microcapsules (from Example 1) | 1.0 |
| BTAC | 2.9 |
| Cetearyl Alcohol | 4.0 |
| DC5 7134 | 2.5 |
| Perfume | 1.0 |
| EDTA | 0.1 |
| Potassium Chloride | 0.1 |
| Water | Up to 100% |

### Example 15 - Skin Body Wash

Table 17 gives a skin body wash composition incorporating the PU microcapsules of Example 1.

**Table 17**

| **Composition** | **% w/w** |
|---|---|
| PU Microcapsules (from Example 1) | 1.0 |
| Carbopol Aqua SF-1 | 1.2 |
| SLES 1EO | 11.0 |
| Cocamide MEA | 1.0 |
| EDTA | 0.05 |
| PPG-9 | 0.1 |
| NaCl (25% solution) | 1.0 |
| Perfume | 1.0 |
| NaOH (50% solution) | 0.01 |
| Water | Up to 100% |

Examples 9 to 15 may alternatively be formulated using the HPC grafted microcapsule from Example 6.

## Claims

1. A composition including a surfactant or cationic material and at least 0.01 wt% of microcapsules containing a benefit agent inside a polyurea shell **characterised in that** the polyurea has a nonionic polysaccharide deposition polymer covalently bonded to it.

2. A composition according to claim 1 which is a laundry detergent composition comprising at least 5 wt% anionic surfactant.

3. A composition according to claim 1 which is a skin cleansing composition or hair shampoo composition comprising at least 2 wt% surfactant.

4. A composition according to claim 1 which is a hair conditioner composition comprising at least 1 wt% cationic material.

5. A composition according to any preceding claim further comprising at least 0.1 wt% perfume.

6. A composition according to any preceding claim in which the deposition polymer is a nonionic polysaccharide selected from the group consisting of mannan, glucan, glucomannan, xyloglucan, hydroxyalkyl cellulose, dextran, galactomannan and mixtures thereof.

7. A composition according to claim 6 in which the deposition polymer is selected from the group consisting of xyloglucan, galactomannan, dextran and hydroxypropyl cellulose.

8. A composition according to claim 7 in which the deposition polymer is xyloglucan or hydroxypropyl cellulose.

9. A composition according to any preceding claim in which the weight ratio of deposition polymer to polyurea shell lies in the range 1:500 to 1:2.

10. A composition according to any preceding claim in which the nonionic polysaccharides have a molecular weight Mw in excess of 40 kDa.

11. A composition according to any preceding claim comprising from 1 to 30 wt% polyurea shell.

12. A composition according to any preceding claim in which the deposition polymer levels are from 0.1 to 10 wt%, based on microcapsule weight.

13. A composition according to any preceding claim in which the polyurea is formed by the reaction of poly-isocyanate and diamine.

14. A method of making the microcapsule for use in the composition according to any preceding claim wherein the nonionic polysaccharide is added at least 15 minutes after commencement of curing of the polyurea.

## Patentansprüche

1. Zusammensetzung, die ein Tensid oder ein kationisches Material und mindestens 0,01 Gew.-% Mikrokapseln, die innerhalb einer Polyharnstoffhülle ein Vorteilsmittel enthalten, einbezieht, **dadurch gekennzeichnet, dass** der Polyharnstoff ein nicht-ionisches Polysaccharidabscheidungspolymer, das an ihn kovalent gebunden ist, aufweist.

2. Zusammensetzung nach Anspruch 1, welche eine Waschmittelzusammensetzung darstellt, die mindestens 5 Gew.-% anionisches Tensid umfasst.

3. Zusammensetzung nach Anspruch 1, welche eine Hautreinigungszusammensetzung oder eine Haarshampoozusammensetzung darstellt, die mindestens 2 Gew.-% Tensid umfasst.

4. Zusammensetzung nach Anspruch 1, welche eine Haarkonditionierzusammensetzung darstellt, die mindestens 1 Gew.-% kationisches Material umfasst.

5. Zusammensetzung nach irgendeinem vorhergehenden Anspruch, die ferner mindestens 0,1 Gew.-% Parfüm umfasst.

6. Zusammensetzung nach irgendeinem vorhergehenden Anspruch, in welcher das Abscheidungspolymer ein nicht-ionisches Polysaccharid darstellt, das aus der Gruppe ausgewählt ist, die aus Mannan, Glucan, Glucomannan, Xyloglucan, Hydroxyalkylcellulose, Dextran, Galactomannan und Mischungen davon besteht.

7. Zusammensetzung nach Anspruch 6, in welcher das Abscheidungspolymer aus der Gruppe ausgewählt ist, die aus Xyloglucan, Galactomannan, Dextran und Hydroxypropylcellulose besteht.

8. Zusammensetzung nach Anspruch 7, in welcher das Abscheidungspolymer Xyloglucan oder Hydroxypropylcellulose darstellt.

9. Zusammensetzung nach irgendeinem vorhergehenden Anspruch, in welcher das Gewichtsverhältnis des Abscheidungspolymers zur Polyharnstoffhülle in dem Bereich von 1:500 bis 1:2 liegt.

10. Zusammensetzung nach irgendeinem vorhergehenden Anspruch, in welcher die nicht-ionischen Polysaccharide ein Molekulargewicht Mw von mehr als 40 kDa aufweisen.

11. Zusammensetzung nach irgendeinem vorhergehenden Anspruch, die von 1 bis 30 Gew.-% Polyharnstoffhülle umfasst.

12. Zusammensetzung nach irgendeinem vorhergehenden Anspruch, in welcher die Anteile des Abscheidungspolymers von 0,1 bis 10 Gew.-%, bezogen auf das Mikrokapselgewicht, betragen.

13. Zusammensetzung nach irgendeinem vorhergehenden Anspruch, in welcher der Polyharnstoff durch die Umsetzung von Polyisocyanat und Diamin gebildet wird.

14. Verfahren zur Herstellung der Mikrokapsel zur Verwendung in der Zusammensetzung nach irgendeinem vorhergehenden Anspruch, wobei das nichtionische Polysaccharid mindestens 15 Minuten nach Beginn des Aushärtens des Polyharnstoffs hinzugegeben wird.

## Revendications

1. Composition incluant un tensioactif ou une substance cationique et au moins 0,01 % en poids de microcapsules contenant un agent avantageux à l'intérieur d'une enveloppe en polyurée **caractérisée en ce que** la polyurée a un polymère de dépôt polysaccharide non ionique lié à elle de manière covalente.

2. Composition selon la revendication 1 qui est une composition de détergent pour blanchissage comprenant au moins 5 % en poids de tensioactif anionique.

3. Composition selon la revendication 1 qui est une composition de nettoyage pour la peau ou une composition de shampoing pour les cheveux comprenant au moins 2 % en poids de tensioactif.

4. Composition selon la revendication 1 qui est une composition de conditionneur pour les cheveux comprenant au moins 1 % en poids de substance cationique.

5. Composition selon l'une quelconque des revendications précédentes comprenant en outre au moins 0,1 % en poids de parfum.

6. Composition selon l'une quelconque des revendications précédentes dans laquelle le polymère de dépôt est un polysaccharide non ionique choisi dans le groupe consistant en le mannane, le glucane, le glucomannane, le xyloglucane, une hydroxyalkylcellulose, le dextrane, le galactomannane et leurs mélanges.

7. Composition selon la revendication 6 dans laquelle le polymère de dépôt est choisi dans le groupe consistant en le xyloglucane, le galactomannane, le dextrane et l'hydroxypropylcellulose.

8. Composition selon la revendication 7 dans laquelle le polymère de dépôt est le xyloglucane ou l'hydroxypropylcellulose.

9. Composition selon l'une quelconque des revendications précédentes dans laquelle le rapport en poids du polymère de dépôt à l'enveloppe en polyurée est situé dans la plage de 1:500 à 1:2.

10. Composition selon l'une quelconque des revendications précédentes dans laquelle les polysaccharides non ioniques ont une masse moléculaire Mp dépassant 40 kDa.

11. Composition selon l'une quelconque des revendications précédentes comprenant de 1 à 30 % en poids d'enveloppe en polyurée.

12. Composition selon l'une quelconque des revendications précédentes dans laquelle les niveaux de polymère de dépôt sont de 0,1 à 10 % en poids, sur la base du poids des microcapsules.

13. Composition selon l'une quelconque des revendications précédentes dans laquelle la polyurée est formée par la réaction de polyisocyanate et de diamine.

14. Procédé de production de la microcapsule destinée à être utilisée dans la composition selon l'une quelconque des revendications précédentes où le polysaccharide non ionique est ajouté au moins 15 minutes après le commencement du durcissement de la polyurée.
